# EUROPEAN PATENT SPECIFICATION

(11) **EP 1 776 066 B1**
(45) Date of publication and mention of the grant of the patent: **08.02.2012**
(21) Application number: 05764407.2
(22) Date of filing: 30.06.2005
(51) Int. Cl.: A61F 2/06

(54) **STENT HAVING ARCUATE STRUTS**
STENT MIT BOGENFÖRMIGEN STREBEN
STENT POURVU D'ENTRETOISES ARQUÉES

(30) Priority: 02.07.2004 US 584994 P
(43) Date of publication of application: 25.04.2007
(73) Proprietor: Cook Medical Technologies LLC, Bloomington, IN 47404 (US)
(72) Inventor: AMARANT, Paul, D., Davie, FL 33330 (US); OSBORNE, Thomas, A., Bloomington, IN 47401 (US)
(74) Representative: Dunne, Emma Louise
(86) International application number: PCT/US2005/023625
(87) International publication number: WO 2006/014383

(56) References cited:
- EP-A- 0 423 916
- WO-A-01/19285
- WO-A-2004/016200
- US-A- 5 507 771
- US-A- 5 556 413
- US-A- 6 004 347

## Description

### FIELD OF THE INVENTION

The present invention relates to medical devices generally used percutaneously and in particular to stents.

### BACKGROUND

Minimally invasive surgical stent technology has become popular since stents were introduced to the medical device market in the United States in the early 1990s. For more than a decade, stents have proven to provide an excellent means for maintaining vessel patency, and have become widely accepted in the medical field.

By way of background, these stents are configured to be implanted into body vessels when a vessel passageway, which may have become partially narrowed, occluded, or otherwise diseased, needs reinforcement, support, repair, or otherwise improved performance to restore or increase blood flow through that diseased section. The term "passageway" is understood to be any lumen, chamber, channel, opening, bore, orifice, flow passage, duct, or cavity for conveying, regulating, flowing, or moving bodily fluids and/or gases in an animal. As an example, stents have been used in many passageways in a body, including a heart, blood vessel, artery, vein, capillary, bronchiole, trachea, esophagus, aorta, intestine, bile duct, ureter, urethra, fallopian tube, and other locations in a body (collectively, "vessel") to name a few.

Stents come in many different configurations. In general, a stent may comprise a ring, or stack of rings, each ring being formed from struts and apices connecting the struts, whereby the stent defines an approximately tube-like configuration. Furthermore, the stent surface may not define a truly round cylinder if the struts are straight, because the struts follow a straight line from the apex on one end of the strut to the apex on the other end of the strut.

In addition, the stent may assume a collapsed tubular configuration having a smaller diameter and an expanded tubular configuration having a larger diameter. In its collapsed smaller diameter configuration, the stent can be constrained with a delivery system (e.g., a sheath, cannula, catheter, or introducer) that helps to position the stent in the vessel passageway at a desired location. After deployment from the delivery system, the stent expands to its larger diameter.

Expandable stents are normally evaluated according to four performance criteria: the radially outward expansile force that the stent exerts on a vessel's interior surface; the small diameter to which the stent is capable of being compressed for the insertion procedure; the stent's ability to traverse curved passageways; and the stability in not migrating from its originally implanted position. Among their many types, expandable stents may be self-expanding, balloon-expandable, or a combination thereof as when the stent is partially self-expandable and partially balloon-expandable.

Generally stated, a balloon-expandable stent is mounted on an expandable member, such as a balloon, provided on the distal end of a delivery system, such as catheter. In operation, a physician, operator, or other healthcare professional (collectively, "physician") advances the catheter to a desired location within the vessel passageway in a patient, and then withdraws the sheath or other covering member from the stent. Next, inflating the balloon plastically deforms the stent into an expanded condition. After deflating the balloon, the physician removes the catheter from the patient's body.

For a self-expanding stent, the stent is resiliently compressed into a collapsed smaller diameter and carried by the delivery system. Due to its construction and/or material properties, the stent expands to its second, larger diameter upon deployment. In its expanded configuration, the stent exhibits sufficient stiffness such that it will remain expanded in the vessel passageway and exert a radially outward force on the vessel's interior surface.

One particularly useful self-expanding stent is the Z-Stent, which Cook Incorporated introduced and made available to the market, due to the Z-Stent's ease of manufacturing, high radial force, and self-expanding properties. Examples of the Z-stent design are found in United States Patent Numbers 5,507,771; 5,035,706; and 4,580,568. By way of example only, stents have utilized this design for applications involving the bronchioles, trachea, thoracic aortic aneurysms (stent-graft), abdominal aortic aneurisms (stent-graft), intestines (biliary tract), and venous valves. The stent is capable of being compressed, inserted into a sheath, pushed out into the passageway of a vessel, and then self-expanded to help keep the vessel passageway in an open state at the stent location. A few devices using embodiments of the Z-stent design include the self-expanding Zilver® biliary stent, the Zilver® 518 biliary stent, the Zilver® 635 biliary stent, and the Zenith® AAA Endovascular Graft, which are available from Cook Incorporated.

US 6,004,347 discloses a graft assembly for securely positioning a grift at a predetermined location across an abdominal aortic aneurysm. The assembly includes a resilient self expanding anchor that is secured to the distal end of a mesh graft. The anchor is formed from a single wire having a series of zigzag bends, such that when the ends of the wire are attached a tubular anchor is produced. The anchor is further characterized by curved segments between the bends which a create an anchor having a reverse barrel shape. [0009b] WO 01/19285 discloses a stent and valve device assembly for manufacture using suitable biocompatible materials and for placement, preferably percutaneously, into a vascular system.

WO 2004/016200 discloses a valve prosthesis, such as an artificial venous valve, having a support frame and leaf structure comprising one or more leaflets in which the outer edge of each leaflet engages the inner circumference of the bodily passageway along a serpentine path urged against the passageway by an expandable frame, while the inner edges move in response to fluid to restrict retrograde flow. Optionally, one or more elements can extend from the support frame/leaf structure to provide centering support and/or protection from the leaflet adhering to the vessel wall. In one embodiment, the centering support structure comprises a second or third expandable frames attached to and extending from the proximal and/or distal ends of main valve structure and support frame. In another embodiment, one or more support elements extend outward from the valve support frame to engage the vessel wall to provide greater longitudinal stability.

US 5,556,413 discloses an intravascular stent comprising a cylindrical body capable of expansion having end assemblies capable of locking in an expanded state. The end assemblies may have a series of tabs and apertures that interlock and rotate as the stent ends expand to an open position to support a section of vascular or other body lumen. The stent is bio-compatible, may be bio-erodible, and capable of localized drug delivery.

US 5,556,413 discloses an intravascular stent comprising a cylindrical body capable of expansion having end assemblies capable of locking in an expanded state. The end assemblies may have a series of tabs and apertures that interlock and rotate as the stent ends expand to an open position to support a section of vascular or other body lumen. The stent is bio-compatible, may be bio-erodible, and capable of localized drug delivery.

It would be desirable to have an additional choice for stents that includes the advantages of the conventional stents and the modifications, as taught herein, that would provide alternative improved clinical performance.

A stent for implanting in a body vessel is provided. In one embodiment, the device includes at least two circumferential elements each having first and second longitudinal ends. Each circumferential element comprises a plurality (i.e., two or more) of arcuate struts and a plurality of strut connections, adjacent struts being joined end to end by at least one strut connection.

The circumferential elements are stackable into a tubular main body, the tubular main body having a first and second ends and defining a lumen along a longitudinal axis. Preferably, the circumferential elements are moveable between a radially compressed first diameter configuration and a radially expanded second diameter configuration. Pluralities of circumferential elements are disposed along a longitudinal axis of the main body. Each circumferential element comprises a plurality of arcuate struts, spaced arcuately relative to a plane including the longitudinal axis and disposed intermediate the first and second ends and a plurality of strut connections, and one or more link members joining the circumferential elements together, Each link member connects the first longitudinal end of one circumferential element to either the first or second longitudinal ends of a second circumferential element. Each strut may have a first and second end portion corresponding to the first and second ends of the circumferential element whether at least one of the struts is convex whereby an intermediate portion of the strut between the first and second end portions is closer to the longitudinal axis that the end portions, or concave where the intermediate portion is further from the longitudinal axis than the end portions.

A method of maintaining vessel patency is also described. In one example, a method comprises providing a delivery system with a distal end portion containing a compressed stent having first and second longitudinal ends, arcuate struts, and strut connections. The distal end portion is inserted into a body and delivered through the body until the unexpanded stent is located where desired. The stent is deployed from the distal end portion of the delivery system and expanded to a second, larger diameter configuration.

In another examplary method of maintaining vessel patency, a stent comprising a circumferential element is provided. The stent inserted into a distal end portion of a stent delivery catheter. The catheter distal end portion is delivered into an internal region of a body to a location where desired. The stent is deployed from the catheter distal end portion and to the expanded configuration.

In another examplary method of using a stent for implantation in a body vessel, a stent is provided comprising a circumferential element. An endoscope is provided that comprises a flexible distal insertion portion and a working channel having a distal opening. Another step comprises providing an elongate stent delivery catheter having a distal end portion configured to be inserted into the endoscope working channel and further having a stent mounting region configured for detachably and releasably securing the stent in a radially compressed configuration.

### BRIEF DESCRIPTION OF THE DRAWINGS

Figure 1A is a perspective view of a medical device according to one embodiment of the invention having concave struts.

Figure 1B is a perspective view of Figure 1A with an alternative embodiment of the strut connections.

Figure 2A is a perspective view of a medical device according to one embodiment of the invention having convex struts.

Figure 2B is a perspective view of Figure 2A with an alternative embodiment of the strut connections.

Figure 3 is a perspective view of convex struts according to one embodiment of the invention.

Figure 3A is a perspective view of a convex strut spaced arcuately relative to a plane including a longitudinal axis of the stent.

Figure 3B shows another convex strut arcing relative to a longitudinal axis.

Figure 3C is a view of the convex strut according to Figure 3B spaced arcuately relative to a plane including the longitudinal axis.

Figure 4 is a perspective view of concave struts according to another embodiment of the invention.

Figure 4A is a perspective view of a concave strut spaced arcuately relative to a plane including a longitudinal axis of the stent.

Figure 4B shows another concave strut arcing relative to a longitudinal axis.

Figure 4C is a view of the concave strut according to Figure 4B spaced arcuately relative to a plane including the longitudinal axis.

Figure 5A is a perspective view of Figure 1A shown in a schematic partial perspective view of a body vessel.

Figure 5B is a perspective view of Figure 1B shown in a schematic partial perspective view of a body vessel.

Figure 6A is a perspective view of Figure 2A shown in a schematic partial perspective view of a body vessel.

Figure 6B is a perspective view of Figure 2B shown in a schematic partial perspective view of a body vessel.

Figure 7A is a schematic perspective view of a medical device according to a tubular embodiment of the invention having concave struts.

Figure 7B is a schematic partial perspective view of an alternative embodiment of Figure 7A having convex struts.

Figure 8A is a partial view of a sutured link member according to an embodiment of the invention.

Figure 8B is a detailed partial view of an alternative embodiment of a sutured link member.

Figure 8C is a perspective view of a link member according to another alternative embodiment of the invention.

Figure 9A is a perspective view of a strut connection in accordance with one embodiment of the invention.

Figure 9B is a perspective view of an alternative embodiment of the strut connection of Figure 9A.

Figure 10 is a perspective view of an alternative embodiment of a strut connection used with an embodiment of the invention.

Figure 11 is a perspective view of an alternative embodiment of a strut connection.

Figure 12 is a perspective view of an alternative embodiment of a strut connection.

Figure 13 is a perspective view of an alternative embodiment of the strut connection of Figure 12.

Figure 14 is a block diagram illustrating a method of the invention.

### DETAILED DESCRIPTION OF THE EMBODIMENTS

The present invention relates to medical devices and, in particular, to stent devices for implanting into vessel passageways. To promote the principles that help in understanding the invention, the following provides a detailed description of several embodiments of the invention as illustrated by the drawings as well as the language used to describe various aspects relating to the invention. The description is not intended to limit the invention in any manner, but rather serves to enable those skilled in the art to make and use the invention. As used in this specification, the terms comprise(s), include(s), having, has, with, contain(s) and variants of those terms are all intended to be open ended transitional phrases, terms, or words that do not preclude the possibility that other steps or structure may be added.

Embodiments of tubular stents according to the present invention comprise "arcuate" struts, which is more fully described below and illustrated in the figures. Briefly stated, the term "arcuate" is used to indicate that the stents bend (i.e., arc) outwardly or inwardly of a cylindrical envelop-as opposed to being straight and lying on a cylindrical envelope. In one embodiment, an arcuate strut is "concave," while an alternate embodiment may have an arcuate strut that is "convex," where the terms "concave" and "convex" are defined relatively as viewed from within the stent, as more fully explained later. Furthermore, the arcuate struts are joined end-to-end by strut connections and formed into a ring or band (i.e., circumferential elements) to form a tubular shape. The circumferential element may be zigzag, serpentine, undulating, Z-shaped, and the like (collectively, "zigzag"). Stent embodiments may have one circumferential element (N=1), or they may comprise more than one circumferential element (N>1) joined together.

Stents may be made of any suitable biocompatible material, or material that is capable of being made biocompatible such as with a coating, chemical treatment, or the like. Examples of suitable materials include, without limitation, stainless steel, nitinol, titanium, nickel titanium, other shape memory and/or superelastic alloys, polymers, polyether block amide, and composite materials, cobalt-chrome alloys, and amorphous metal alloys. Stainless steel and nitinol are biocompatible and particularly shapeable, although nitinol is less stiff than is stainless steel and, thereby, will not produce the same radial force in a similarly-sized stent. The stent may also be made of a degradable polymer, such as a homopolymer, blend, or copolymer of Poly-alpha Hydroxy Acids (e.g., Polyactic Acid or Polyglycolic Acid), Trimethylene Carbonate, Polycaprolactone, Poly-beta Hydroxy Acids (e.g., Polyhydroxybutyrate, Polyhydroxyvalerate), Polyphosphazines, Polyorganophosphazines, Polyanhydrides, Polyesteramides, Polyorthoesters, Polyethylene oxide, Polyester-ethers (e.g., Polydioxanone), and/or Polyamino acids (e.g., Poly-L-glutamic acid or Poly-L-lysine). The term "material" shall include any one or more material(s) from this list used individually or in combination in a stent embodiment according to the invention.

In addition, a stent can be formed from the material in many different ways. By way of illustration and not by way of limitation, the wire for use as or in a stent or stent component may be formed from extrusion or may be purchased as wire that is commercially available. Then, the wire may be turned or bent to form strut connections, and the wire with strut connections may be placed and shaped between two blocks with complementary curved interfaces corresponding to the desired shape of the arcuate struts. Strut connections and arcuate struts are discussed below. With its strut connections and arcuate struts, the length of wire may then be wrapped around, molded, and shaped onto a mandrel. Alternatively, the stent pattern may be cut from a tube or sheet of material, laser cut, chemical etched, stamped, electric discharge machined, or formed by other known processes.

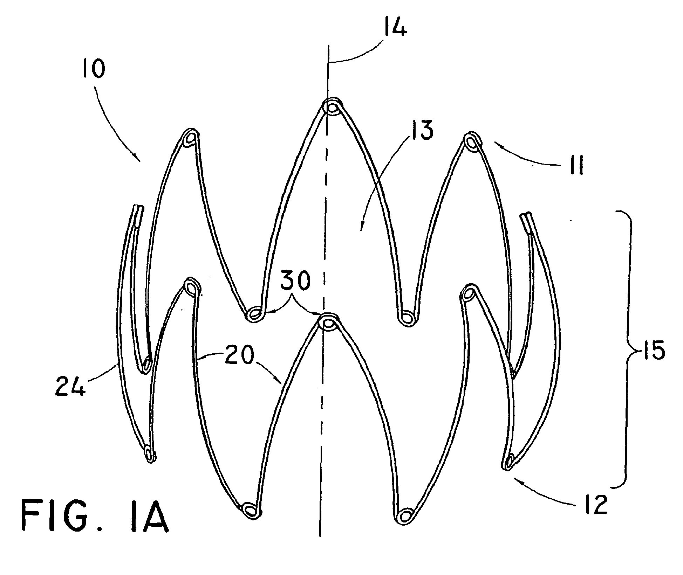

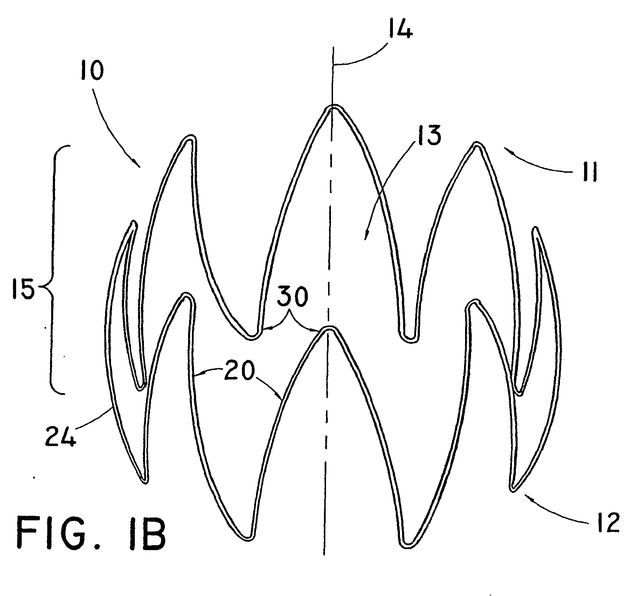

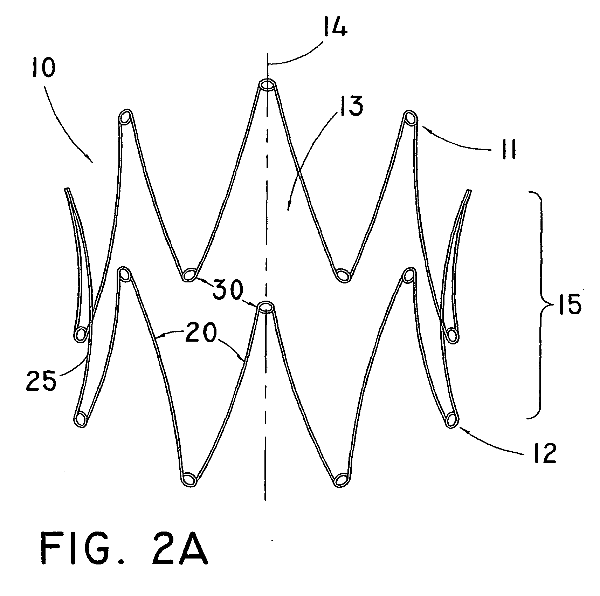

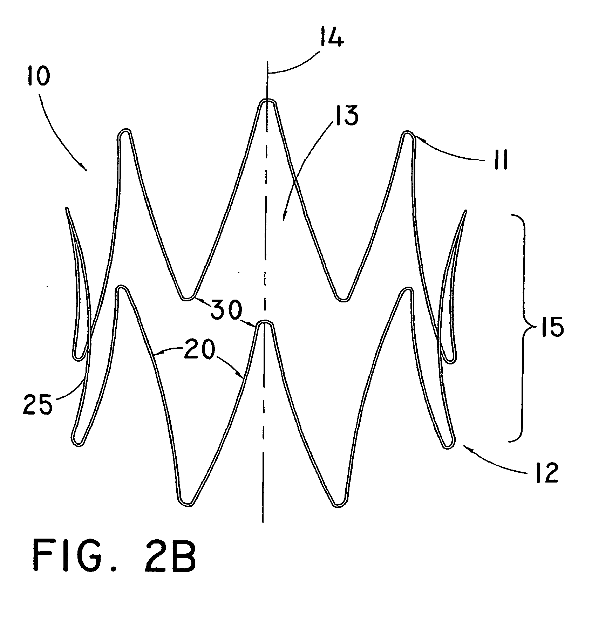

Figures 1A, 1B, 2A, and 2B illustrate several embodiments of stents 10 in accordance with the invention, and show each stent in a radially expanded configuration. The stent 10 has first and second longitudinal ends 11, 12 defining a lumen 13. The terms "first" and "second" as used to describe the embodiments are terms used as a convention that merely distinguishes elements in the embodiment, and not as terms supplying a numerical limit, serial limit, spatial location, or any differences in the ends unless expressly and unequivocally stated otherwise. In one embodiment, for example, the first end 11 may be located at or near a proximal portion of the stent while the second end 12 is located at or near a distal portion, although the first end does not necessarily need to appear before the second end, and the second end does not necessarily appear after the first end. As is conventional, "distal" means the end that is directed or oriented away from the physician when the device is inserted into a patient while "proximal" means the end that is closer to or toward the physician relative to the distal end.

Also, the lumen 13 defines a longitudinal axis 14. The term "longitudinal" and its variants are intended to mean, in a broad sense, running longitudinally (lengthwise). The longitudinal axis 14 need not necessarily be straight, and could in fact curve as in a tangent to a referenced segment or point on or contained within a stent lumen 13. Indeed, given the flexibility of a stent, the "longitudinal" axis may be straight at some portions of the lumen and bent or curved at other portions.

In their expanded configurations and when viewed end-on, a stent 10 generally exhibits a tubular cross-section containing the lumen 13. Furthermore, in the expanded configuration the tubular cross-section of the stent need not be constant between its proximal and distal ends 11, 12, respectively, and may be straight or curved, because the stent is configured for placement in a vessel passageway. In one embodiment, the stent may be modified for a bifurcated vessel or a passageway that brachiates, and the stent has side tubular branches or has a portion that contains two tubular stent bodies side-by-side of equal or unequal length, which is particularly suited in the field of cardiology. Furthermore, the specific dimensions of a particular stent according to the invention will depend on numerous factors, including the type of vessel in which the device is to be implanted, the axial length of the treatment site, the inner diameter of the vessel, the delivery method for placing the stent, and others. Those skilled in the art can determine an appropriately sized and dimensioned stent based on these various factors.

In essence, the body of a stent contains one or more circumferential elements 15. Generally stated, a circumferential element comprises strut connections 30 at the first and second ends 11, 12, respectively, and struts 20 intermediate the first and second ends 11, 12 of the stent 10. The term "intermediate" is intended to mean between, though not necessarily equidistant to, the distal most tip of the second end 12 and the proximal most tip of the first end 11.

### STRUTS

As generally stated, a stent 10 includes at least one circumferential element 15. Figures 1A through 2B show views of embodiments each having one (N=1) circumferential element 15, whereby the circumferential elements (shown in an expanded state) comprise struts 20 and strut connections 30. More particularly, a circumferential element comprises, for example, a "zigzag" pattern (as previously described) that contains a series of concave 24 or convex 25 struts, whereby strut connections 30 (described later) join adjacent struts 20. Figures 1A and 1B show circumferential elements 15 according to two illustrative embodiments whereby the struts 20 are concave 24. In contrast, Figures 2A and 2B show circumferential elements 15 according to two illustrative embodiments whereby the struts 20 are convex 25.

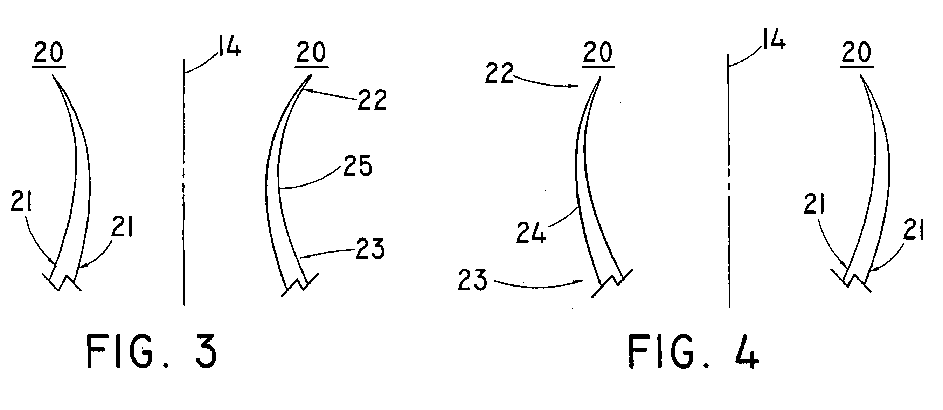

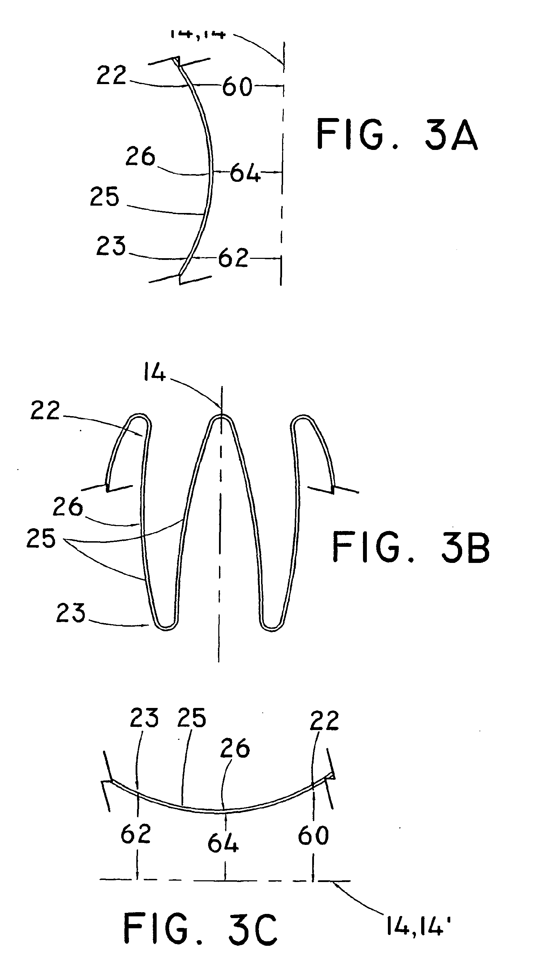

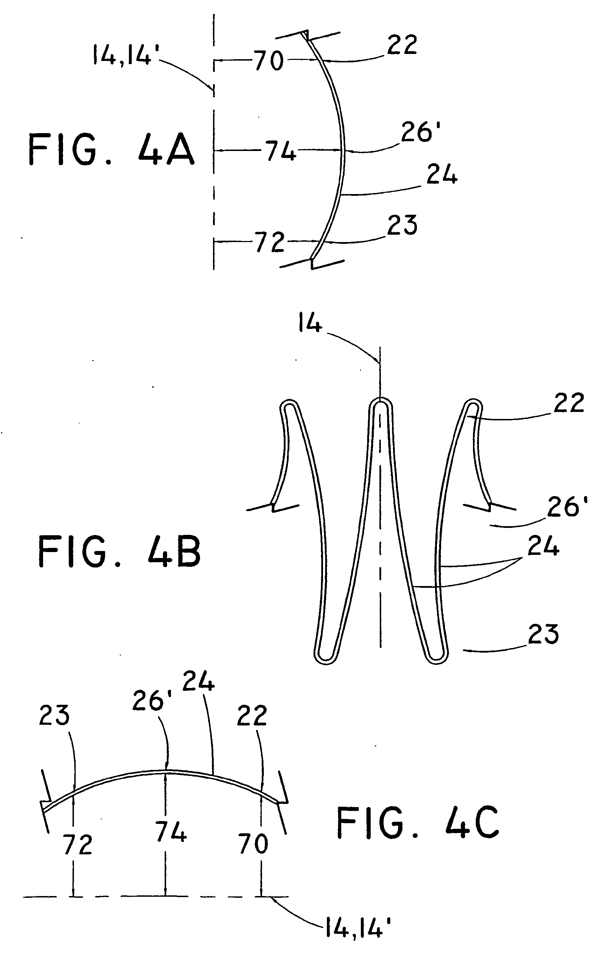

Referring to an arcuate strut 20 as concave 24 or convex 25 is largely a matter of perspective as well as the frame of reference used in describing embodiments according to the invention. Figures 3 and 4 show, for example, enlarged schematic views of embodiments of struts 20, which optionally can be utilized in Figures 1A, 1B, 2A, and 2B, in order to assist in explaining concave and convex as used in this specification to describe embodiments of the invention, and not as any lexicographic definition. As shown in Figures 3 and 4, a strut 20 comprises a wire member 21, and the strut has first and second end portions 22, 23 corresponding, respectively, to proximal and distal ends 11, 12 of the stent 10. The wire member 21 arcs (e.g., follows an arc-shaped course) intermediate the first and second end portions 22, 23, and the arc may be either concave 24 or convex 25. The longitudinal axis 14 helps to provide a frame of reference for labeling a strut as either concave or convex, whereby Figure 3 depicts a convex strut 25 while Figure 4 depicts a concave strut 24 in reference to the longitudinal axis 14 of those figures. The arcuate struts 20 are spaced arcuately relative to a plane 14' including the longitudinal axis 14. In other words, the struts bend (i.e., arc) outwardly or inwardly of a cylindrical envelop-as opposed to being straight and lying on a cylindrical envelope.

Figures 3A, 3B, and 3C illustrate convex struts 25 spaced arcuately relative to a plane 14' including the longitudinal axis 14. Figure 3A shows a convex strut 25 according to the embodiment in Figure 3, whereby the first and second end portions 22, 23, respectively, will be spaced at distances 60, 62, respectively, from the plane 14' greater than the distance 64 from an intermediate portion 26 of the strut 25 to the plane 14'. The distances compare as follows: 64 < 60 and 64 < 62. In other words, the intermediate portion 26 in a convex strut 25 is closer to the longitudinal axis 14 than the first end second end portions 22, 23 are to the longitudinal axis 14. Figure 3B shows another convex strut 25 arcing relative to the longitudinal axis 14. Expanded struts 20 may not be parallel to the longitudinal axis 14 but instead be angled relative to the axis 14 when viewed radially in order for the struts 20, when joined end-to-end by strut connections, to encircle the axis and to form a tubular cross-section containing a lumen. In order to better explain an arcuate strut, therefore, it should be understood that a plane 14' along the length and width of the stent (e.g., generally bisects the stent lengthwise) the longitudinal axis 14 such that the arcuate struts are spaced arcuately relative to the plane 14', whereby the intermediate portion 26 in a convex strut 25 is closer to the plane 14' than the first and second end portions 22, 23, respectively, are to the plane 14'. Figure 3C shows Figure 3B from the perspective of the plane 14', whereby the first and second ends 22, 23 are spaced arcuately at distances 60, 62 relative to the plane 14', and the intermediate portion 26 spaced arcuately at a distance 64 relative to the plane 14'.

Figures 4A, 4B, and 4C illustrate concave struts 24 spaced arcuately relative to a plane 14' including the longitudinal axis 14. Figure 4A shows a concave strut 24 according to the embodiment in Figure 4, whereby the first and second end portions 22, 23, respectively, will be spaced at distances 70, 72, respectively, from the plane 14' greater than the distance 74 from an intermediate portion 26' of the strut 24 to the plane 14'. The distances compare as follows: 74 < 70 and 74 < 72. In other words, the intermediate portion 26 in a concave strut 24 is farther from the longitudinal axis 14 than the first and second end portions 22, 23 are to the longitudinal axis 14. Figure 4B shows another concave strut 24 arcing relative to the longitudinal axis 14. As previously explained, expanded struts 20 may be angled relative to the longitudinal axis 14 when viewed radially. Therefore, a plane 14', as previously described, includes the longitudinal axis 14 such that the arcuate struts are spaced arcuately relative to the plane 14'. In the case of concave struts 24, the first and second and portions 22, 23, respectively, are closer to the plane 14' than the intermediate portion 26 is to the plane 14'. Figure 4C shows Figure 4B from the perspective of the plane 14', whereby the first and second ends 22, 23 are spaced arcuately at distances 70, 72 relative to the plane 14', and the intermediate portion 26' spaced arcuately at a distance 74, relative to the plane 14'.

A strut may vary in length depending on the overall desired length of the expanded circumferential element 15. In addition, the cross-sectional profile of a strut may be, for example only and not by way of limitation, a generally solid tubular shape or a flat rectangular design.

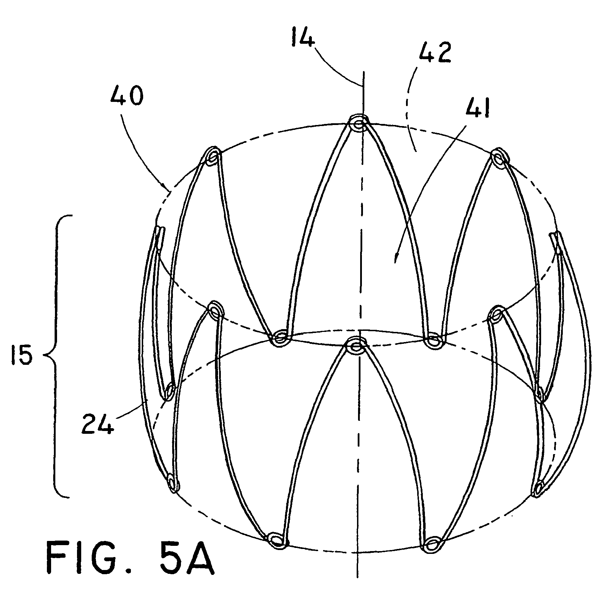

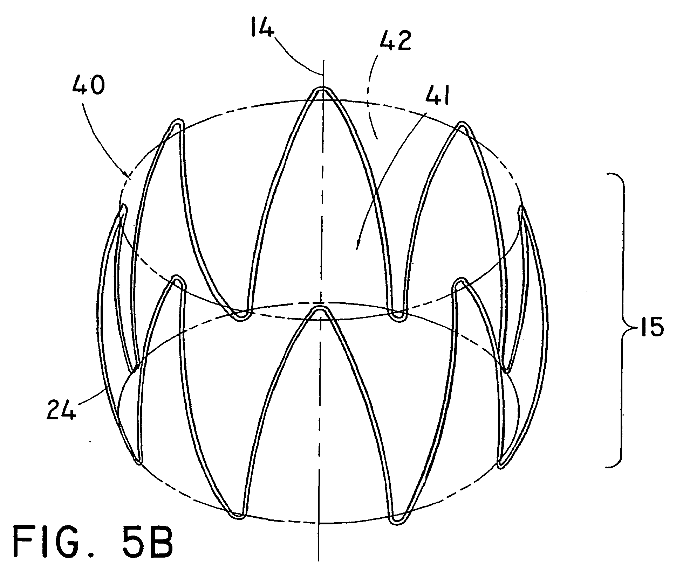

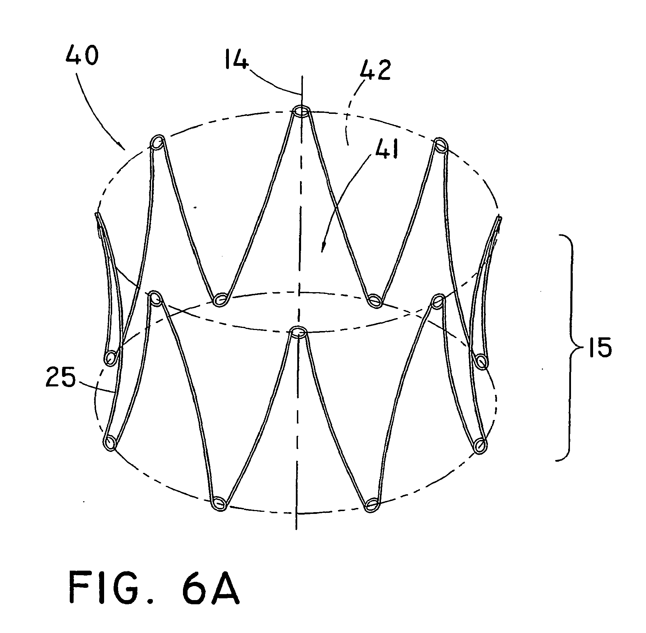

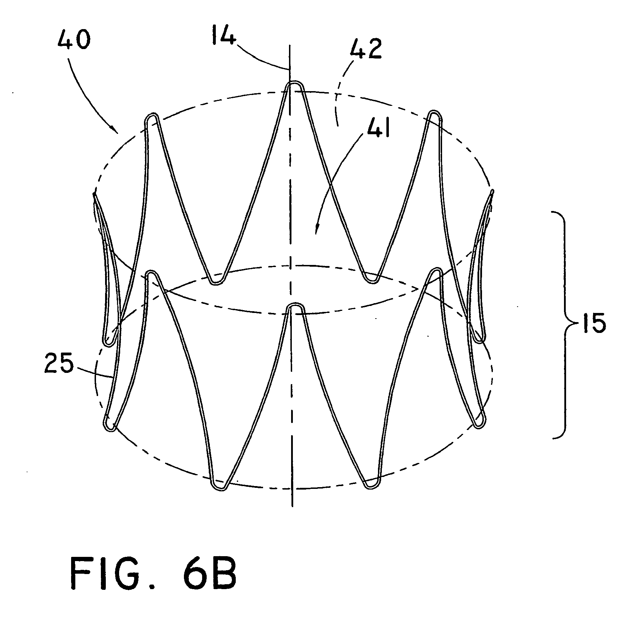

Turning to Figures 5A, 5B, 6A, and 6B, alternative embodiments of circumferential elements 15 are shown. These Figures show a perspective of a passageway 41 of a vessel 40. Furthermore, Figures 5A through 6B provide a perspective view of the circumferential element 15 as deployed such that the circumferential element 15 has radially expanded in an abutting relationship to the interior surface 42 of the vessel passageway. Figures 5A and 5B show embodiments with a circumferential element 15 having concave struts 24, while Figure 6A and 6B depict embodiments with a circumferential element having convex struts 25.

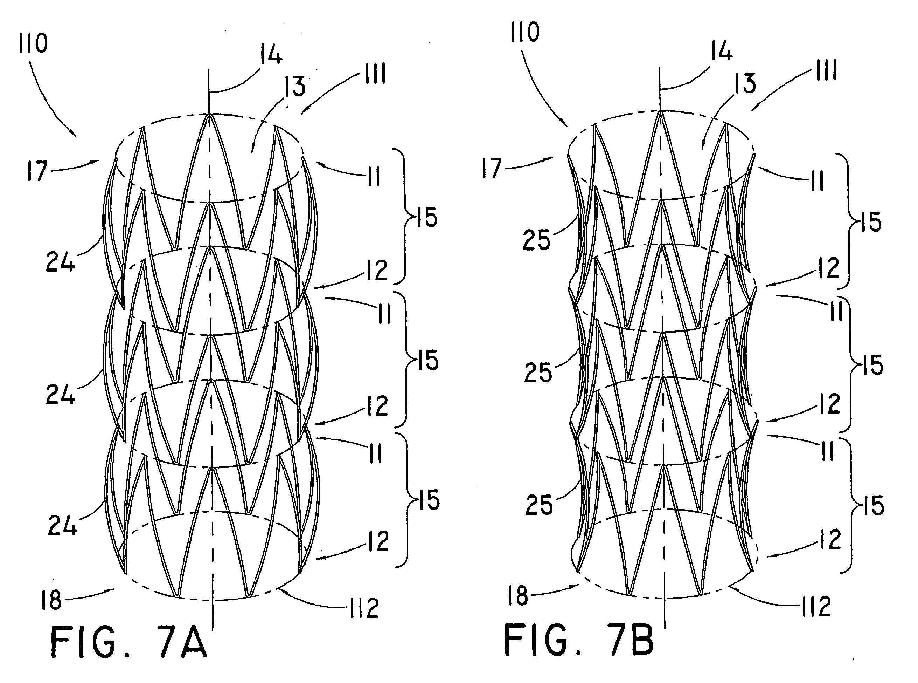

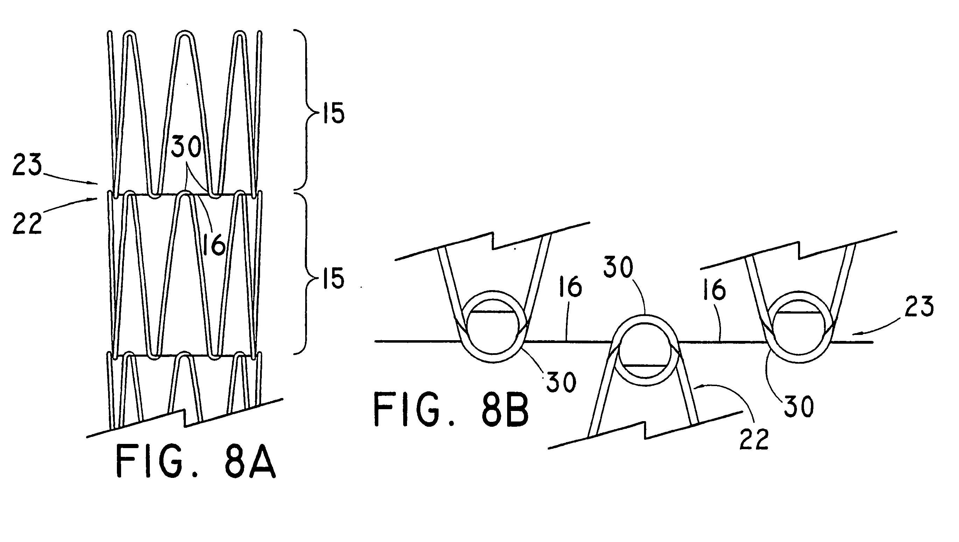

As shown in Figures 7A and 7B, embodiments may have more than one circumferential element 15 (N>1). For example, circumferential elements 15 may be stackable via link members 16 (e.g., Figs. 8A-8C) into a main body 110 that is tubular and having first and second ends 111, 112. The circumferential elements 15, as previously described, have first and second longitudinal ends 11, 12, respectively. The tubular main body 110 first and second ends 111, 112 defining a lumen 13 along a longitudinal axis 14, whereby the lumen is in communication with openings 17, 18, respectively, at or near the first and second ends 111, 112, respectively. The main body has, on the one hand, a radially compressed first diameter configuration (not shown) that is self-expandable, balloon-expandable, or a combination thereof, and has, on the other hand, a radially expanded second diameter configuration. The circumferential elements have a "zigzag" pattern (previously described) containing a series of concave (Figure 7A) or convex (Figure 7B) struts interconnected by strut connections as in Figures 1A-2B, 5A-6B, and 9A-13.

For instance, Figure 7A is an embodiment of a main body 110 having concave struts 24 in a radially expanded second diameter configuration. The main body of Figure 7A has more than one circumferential element 15 joined by a link member 16 (e.g., Figs. 8A-8C) in order to form a column of circumferential elements 15 with concave struts 24. Figure 7B is an embodiment of a main body 110 having convex struts 25 in a radially expanded second diameter configuration, whereby more than one circumferential element 15 is joined by a link member 16 (e.g., Figs. 8A-8C) to form a column of linked circumferential elements 15 with convex struts 25.

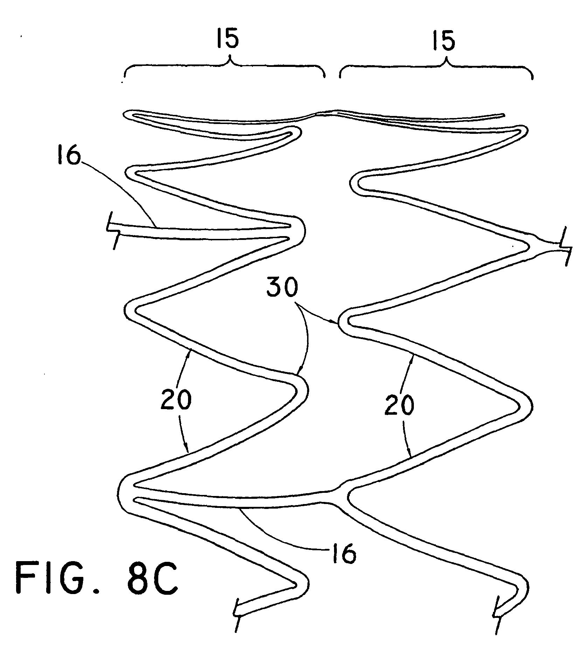

Figures 8A, 8B, and 8C are alternative embodiments of link members 16 for connecting circumferential elements 15 to form a column of linked circumferential elements 15 as in Figures 7A and 7B, by way of illustration only and not be way of limitation. Thus, a strut connection 30 located at a first end portion 22 corresponding to a distal end 12 of a circumferential element 15 may be secured to a strut connection 30 located at a second end portion 23 corresponding to a proximal end 11 of an adjacent circumferential element 15. In Figures 8A and 8B, the link member 16 comprises a suture, whereby the suture is threaded through the strut connections (described below) of one circumferential element to corresponding adjacent strut connections of a second circumferential element. Figure 8C utilizes a link member 16 formed from material, whereby the link member 16 connects a portion of a strut (or strut connection) of one circumferential element 15 to a portion of a strut (or strut connection) of a second circumferential element 15. Indeed, the entire stent can be formed integral as, for example, by laser cutting a tube. Alternatively, a link member or struts themselves on adjacent circumferential elements may be joined by sutures, welds, solder, glue, or additional wires.

Against the foregoing backdrop, an aspect of the utility of the arcuate struts is that concave and convex struts 24, 25, respectively, produce improved clinical results in the vessel passageway. An embodiment is pressed into the interior surface 42 of a vessel passageway 41 so as to not interfere with the fluid flow through the passageway. Also, the stent, being pressed into the interior surface 42 of the vessel passageway, may eventually be covered with endothelial cell growth that further minimizes fluid flow interference.

In addition, concave and convex struts provide additional unique results. A circumferential element 15 or a column of circumferential elements 15 having convex struts 25 (as shown in Figures 2A, 2B, 6A, 6B, and 7B) provides improved tacking characteristics to prevent stent migration within the vessel passageway. In particular, convex struts 25 provide discrete points of attachment to the interior surface 42 of the vessel passageway 41 to tack up and better hold the stent in place and minimize migration, which is beneficial in many applications, such as bronchiole or tracheal applications, for example. Thus, when a stent with convex struts 25 is expandably deployed in the passageway 41 of a vessel 40, the strut connections 30 (discussed below) located at the first and second ends 22, 23, respectively, of the struts make good contact with the interior vessel wall.

A circumferential element 15 or a column of circumferential elements 15 having concave struts 24 (as shown in Figures 1A, 1B, 5A, 5B, and 7A) provides improved radial force and decreases trauma to the interior surface 42 of the passageway 41 of a vessel 40, which is preferred in circulatory applications. Thus, an expanded stent having concave struts 24 more closely models a cylinder than does a conventional stent, and a cylindrical stent is advantageous in the case of large stents, especially stent grafts. For instance, stented grafts (which are commonly used to bypass aneurysms) preferably form a seal against the interior vessel wall 42 in order to isolate the aneurysm from blood pressure. This challenge is addressed today by significantly oversizing the stent graft, which essentially stretches the vessel wall to the point of making a seal all the way around the periphery of the stent. By giving struts a concave arc shape 24, each strut provides an arcuate attachment to the interior surface 42 of the vessel passageway 41 and better fits the curve of the vessel wall, thereby leaving fewer and smaller gaps between the graft material and the vessel wall into which blood can leak, and without oversizing the stent or overstretching the vessel wall.

### STRUT CONNECTIONS

Arcuate struts 20 for a circumferential element 15 are joined together end-to-end by strut connections 30 in alternating fashion. Otherwise stated, a strut connection 30 links an end portion 22 or 23 of one strut and an end portion 22 or 23 of a second strut. Therefore, when formed into a ring-like tubular structure, the successively joined struts and strut connections together form the circumferential elements 15 depicted in Figures 1A through 2B and in Figures 5A through 7B and formed at first and/or second longitudinal ends 11, 12 of the stent 10, although other structure may be added. The words "formed at" an end include a location that is at or within a short distance of the distal most (or proximal most, respectively) tip of that end.

More specifically, strut connections 30 comprise a bend of material (as described above) having a cross-sectional profile that, for example only and not by way of limitation, may be generally tubular shaped or have a rectangular design. Furthermore, strut connections 30 are capable of being compressible to a compressed configuration. In a self-expandable stent, strut connections may be biased to expand radially to an expanded configuration. When expanded (via a balloon, self-expanding properties, or a combination thereof) to their radially expanded configuration, the strut connections 30 of a circumferential element 15 provide discrete points of attachment.

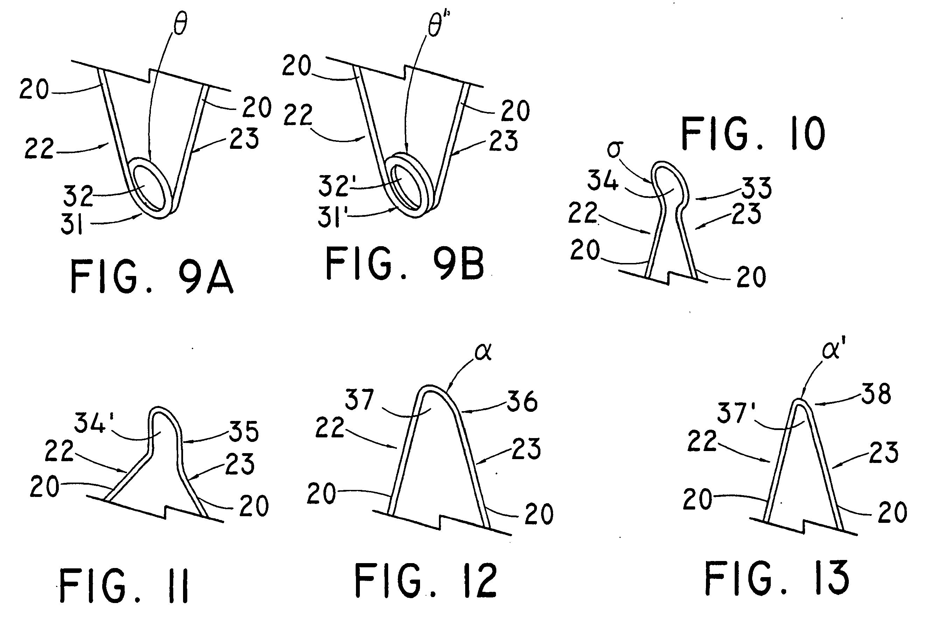

Figures 9 through 13 show enlarged views of embodiments of strut connections 30 in their expanded configurations. When compressed, a strut connection 30 may be expanded by a balloon. Alternatively, a strut connection 30 may be biased to an expanded configuration due to the material's elastic memory, which results in a spring-like effect when a stent has been compressed to a collapsed diameter configuration. Furthermore, the strut connection in Figures 9A and 9B are a "coiled" structure. The term "coiled" and variants thereof, as used to describe embodiments of the invention and not used as any lexicographic definition, means that a length of the material comprises at least one turn as when the length of material is rolled or twisted into a shape that is looped, circular, spiral, bent, curled, or headed.

With reference to Figure 9A, the coiled strut connection 31 comprises a turn that includes about 1½ turns of wire, although the turns may range from about 1 ¼ turns to about 1 ¾ turns. The turns from the strut connection 31 connect a first end portion 22 of a first strut and a second end portion 23 of a second strut. The strut connection 31, due to its turn, forms a closed eyelet 32 that appears as a 360 degree bend or more. In the embodiment illustrated in Figure 9A, the strut connection 31 goes through a turn θ in the range of about 480 and 540 degrees, and is shown in Figure 9A at about 520 degrees, and forms a closed eyelet 32. The strut connection 31, due to its turn, distributes the stress over a longer length of strut than would be the case in a simple sharp bend. This is an advantage for stents that utilize large diameter struts requiring high radial force. Such a case would be, for instance, an esophageal stent in the case of tumor invasion. Figure 9B is an alternative embodiment of Figure 9A, whereby the strut connection 31' goes through an additional turn θ' of roughly 360 degrees and forms a closed eyelet 32'.

Figure 10 illustrates a strut connection 33 comprising an open-ended ogee arch-like compound-curved structure generally having a turn σ that resembles a head-neck-shoulder design (a "head strut connection"). The head strut connection 33 connects a first end portion 22 of a first strut and a second end portion 23 of a second strut. The bend of material of a head strut connection 33 extends through a turn σ that is in the range of about 200 and 270 degrees to form an open eyelet 34. The strut connection 33 distributes stress over a longer length of strut than would be the case in a sharp bend, but distributes stress over a shorter length of strut than a strut connection 31. By ending the turn σ at less than 360 degrees, the strut connection 33 occupies less space and can be compressed into a smaller delivery system.

In addition, strut connections of Figures 9A, 9B, and 10 have convenient eyelets 32, 32', and 34, respectively, in which suture material can be laced or tied in order to form a stent having a stackable (column) configuration of circumferential elements. Thus, the suture material may be threaded though eyelets 32 (Figure 8A) or tied to eyelets 34 (Figure 8B) to form the link members 16 of Figures 8A and 8B. In the embodiment of a stent 10 that comprises a column of circumferential elements as in Figures 7A and 7B, a link member 16 may at properly spaced intervals replace a strut connection 30 so as to join adjacently positioned struts 20 as shown in Figure 8C.

Even in an embodiment having a single circumferential element 15, the embodiment may include a strut connection position whereby a strut connection occasionally may be skipped or replaced, as when adjacent strut ends are joined by other means. For example, ends 22 or 23 of two adjacent struts may be joined by a radiopaque gold marker for the purpose of visualization during placement of the stent. Alternatively, the ends 22 or 23 of adjacent struts may be joined by any other suitable means, such as sewing, adhesives, wires, weld, solder, glue, chemical cross-linking, heat source, light source, radiofrequency, laser, or other energy source.

The strut connections 31, 31', and 33 of Figures 9 and 10 are capable of compression and expansion without plastic deformation. In contrast, a V-shaped strut connection would be limited by mechanical failure and plastic deformation, whereby compression results in work hardening, kinking, or even snapping.

Alternative embodiments of coiled strut connections 35, 36, 38 are shown with bends in Figures 11 through 13. Figure 11 shows a strut connection 35 that has a "U-shaped" or tombstone profile and open eyelet 34', and connects a first end portion 22 of a first strut and a second end portion 23 of a second strut. The strut connection 38 of Figure 12 has a modified, rounded V-shaped vertex σ' having an open eyelet 37' and joining first and second end portions 22, 23 of first and second struts, respectively. Figure 13 illustrates a strut connection 36 having a modified, sharp V-shaped vertex σ" profile having an open eyelet 37 and connecting first and second end portions 22, 23 of first and second struts, respectively.

### COMPRESSED STENT

Strut connections in the compressed (collapsed) configuration compete for and take up space. Therefore, a medium-sized or a larger introducer system might be preferred to accommodate a stent, because the strut connections essentially have to occupy the same space in the introducer system. A medium-sized or larger delivery system is fine for esophageal stenting, as one non-limiting example. However, it may be difficult to deliver a larger stent percutaneously into a vessel passageway having a smaller inner diameter, as in the vascular system. As a result, when the need to have a smaller introducer system becomes important, such as for the introduction of stents into the coronary arteries or cranial vessels, the strut connections are preferably smaller so as to occupy even less space.

The actual ratio of expanded to collapsed size is a function of the material elasticity or how much deflection the material can absorb before being plastically deformed. For example, a strut in AISI type 304 stainless steel might allow a 15 or 20% deflection while Nitinol in the superelastic condition might allow a 30 to 40 % deflection. It is also a function of the number of struts around the periphery of the stent.

The stents are formed of wire arranged in a closed configuration that includes a series of struts that are joined by an equal number of joints. The ends of the wire can be closed in a variety of ways, including the use of a sleeve which is welded or crimped against the ends of the wire to produce a continuous or endless configuration.

### METHODS

A method of using arcuate stents for implantation in a body vessel in order to maintain vessel patentcy is described.

In practice, the stent is compressed into a reduced first diameter size for insertion into, and possible removal from, a body passageway. After being properly positioned within a body passageway, the stent is allowed to expand to an expanded second diameter shape.

In an alternative exemplary method, a sleeve is utilized with the stent for implantation. The stent compressed into a reduced first diameter size for insertion into the sleeve. The sleeve is positioned in a body passageway and removed so as to allow the stent to expand to an expanded second diameter shape. The sleeve may be made of any suitable material (natural, synthetic, or combination thereof) such as a plastic that is pliable, strong, resilient, elastic, and flexible. The sleeve material should be biocompatible, or should be able to be made biocompatible, such as by coating, chemical treatment, or the like.

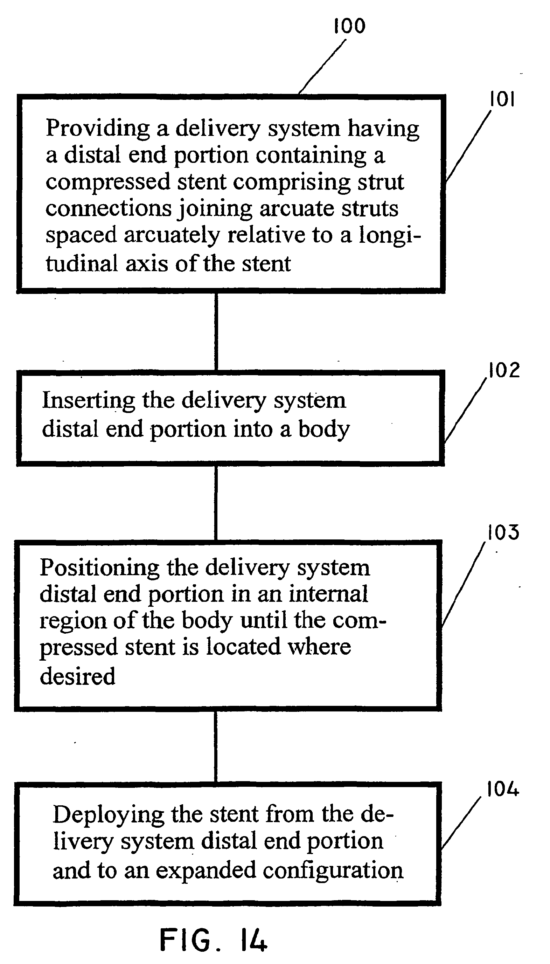

Figure 14 shows a method 100, that comprises providing a delivery system (such as a catheter, endoscope, other endoscope accessory, or the like) with a distal end portion containing a compressed stent having a circumferential element 15 moveable between radially compressed and radially expanded configurations, the circumferential elements 15 further having first and second longitudinal ends 11, 12, respectively, and a longitudinal axis 14 and arcuate struts 20 being joined at the first and second ends by strut connections 30, 31, 31', 33, 35, 36, 38 and where the struts in the expanded configuration are spaced 60, 62, 64, 70, 72, 74 arcuately relative to a plane 14' that includes the longitudinal axis (step 101). The distal end portion is inserted into a body through a mouth, orifice, or incision (step 102). The distal end portion is positioned in an internal region of a body until the compressed (unexpanded stent) is located where desired (step 103). The stent is deployed from the distal end portion of the delivery system and to an expanded configuration (step 104).

Another method of maintaining vessel patency comprises providing a stent having a circumferential element 15 moveable between radially compressed and expanded configurations, the circumferential element 15 further having first and second longitudinal ends 11, 12, respectively, and a longitudinal axis 14 and arcuate struts 20 being joined at the first and second ends by strut connections 30, 31, 31', 33, 35, 36, 38 and wherein the struts in the expanded configuration are 60, 62, 64, 70, 72, 74 arcuately relative to a plane 14' that includes the longitudinal axis. The stent while in a compressed configuration is inserted into a distal end portion of a stent delivery catheter. The distal end portion of the stent delivery catheter is delivered into an internal region of a body until the compressed stent is located where desired. The stent is deployed from the distal end portion of the delivery system and to the expanded configuration.

In another method of using a stent for implantation in a body vessel, a stent is provided in one step, whereby the stent comprises a circumferential element 15 moveable between radially compressed and radially expanded configurations, the circumferential element 15 further having first and second longitudinal ends 11, 12, respectively, and a longitudinal axis 14 and arcuate struts 20 being joined at the first and second ends by strut connections 30, 31, 31', 33, 35, 36, 38 and where the struts in the expanded configuration are spaced 60, 62, 64, 70, 72, 74 arcuately relative to a plane 14' that includes the longitudinal axis. In another step, an endoscope is provided that comprises a flexible distal insertion portion with a distal light and lens for visualizing the interior of an internal region of a body and a working channel having a distal opening for passing a medical device into the observation field and working space of the endoscope, and further configured to provide suction for drawing target tissue toward the distal end of the flexible distal insertion portion. Another step comprises providing an elongate stent delivery catheter having a distal end portion configured to be inserted into the endoscope working channel, the distal end portion further having a stent mounting region configured for detachably and releasably securing the stent in a radially compressed configuration. In addition to those steps, one step comprises positioning the endoscope distal insertion portion to a designated site within a vessel for implantation. Still another step comprises disposing the compressed stent within the catheter stent mounting region, then in another step inserting the catheter distal end portion into the endoscope working channel, and in another step deploying the stent from the catheter distal end portion wherein the circumferential element first end 11 is expelled from the catheter distal end portion to a radially expanded configuration at the designated site and wherein the circumferential element second end 12 is expelled from the catheter distal end portion to a radially expanded configuration at the designated site. One the disposing, inserting, and deploying steps with a second stent having a circumferential element 15 moveable between radially compressed and radially expanded configurations, the circumferential element 15 further having first and second longitudinal ends 11, 12, respectively, and a longitudinal axis 14 and arcuate struts 20 being joined at the first and second ends by strut connections 30, 31, 31', 33, 35, 36, 38 and where the struts in the expanded configuration are spaced 60, 62, 64, 70, 72, 74 arcuately relative to a plane 14' that includes the longitudinal axis. A further step comprises withdrawing the endoscope containing catheter from the vessel. Steps for the forgoing methods need not be performed sequentially. For example only, an endoscope, stent, and catheter may be provided in any order.

It is intended that the foregoing detailed description of the medical devices and method of maintaining vessel patency be regarded as illustrative rather than limiting. It should be understood that it is the following claims, including all equivalents that are intended to define the scope of this invention. Terms are to be given their reasonable meaning and like terms may be used interchangeably in the broadest sense to achieve a particular result. Therefore, the embodiment of any figure and features thereof may be combined with the embodiments depicted in other figures. Other features known in the art and not inconsistent with the structure and function of the present invention may be added to the embodiments.

While particular elements, drawings, embodiments, and applications of the present invention have been shown, illustrated, and described, it will be understood, of course, that the invention is not limited thereto since modifications may be made by those skilled in the art, particularly in light of the foregoing teachings. Therefore, it is therefore contemplated by the appended claims to cover such modifications as incorporate those features which come within scope of the invention, as defined in the claims.

## Claims

1. A stent (10) for implantation in a body vessel, the stent comprising:
at least two circumferential elements (15) each having first and second longitudinal ends (11, 12) and a longitudinal axis (14),
a plurality of strut connections (30, 31, 31', 33, 35, 36, 38) formed at the first end;
a plurality of strut connections (30, 31, 31', 33, 35, 36, 38) formed at the second end;
a plurality of arcuate struts (20) spaced arcuately (60, 62, 64, 70, 72, 74) relative to a plane including the longitudinal axis and disposed intermediate the first and second ends;
wherein the adjacent struts are joined end-to-end by at least one strut connection, and wherein said circumferential elements are joined by one or more link members (16) to form a tubular main body (110) **characterised in that**, each link member connects the first longitudinal end of one circumferential element to either the first or second longitudinal end of a second circumferential clement.

2. The device of claim 1 wherein each strut has a first and second end portion (22, 23) corresponding to the first and second ends of a circumferential element wherein at least one of the struts is convex (25) whereby an intermediate portion (26) of the strut between. the first and second end portions is closer to the longitudinal axis than the end portions.

3. The device of claim 1 wherein each strut has a first and second end portion (22, 23) corresponding to the first and second ends of a circumferential element wherein at least one of the struts is concave (24) whereby an intermediate portion of the strut between the first and second end portions is farther from the longitudinal axis than the end portions.

4. The device of any preceding claim wherein the tubular main body has a longitudinal axis (14) and first and second longitudinal ends (111, 112) and defines a lumen (13) in communication with openings (17, 18) at the first and second ends, the main body having a radially compressed first diameter configuration and a radially expanded second diameter configuration.

5. The device of claim 4, wherein the expanded configuration comprises a Z-shaped zigzag pattern.

6. The device of any preceding claim wherein the circumferential element (15) or tubular main body (110) is balloon-expandable.

7. The device of any of claims 1 to 5 wherein the circumferential element (15) or a tubular main body (110) is self-expandable.

8. The device of any of claims 1 to 7 wherein the body (110) is partially self expandable and partially balloon-expandable.

9. The device of any preceding claim wherein one of the strut connections comprises a head strut connection (33).

10. The device of any preceding claim wherein at least one of the strut connections comprises a coiled structure (31, 32).

11. The device of any preceding claim wherein at least one of the strut connections comprises a bend (35, 36, 38) of material which acts as a discrete attachment point for securing the stent to the vessel wall.

12. The device of any preceding claim wherein one or more of the plurality of strut connections have a closed eyelet (32, 32').

13. The device of any preceding claim wherein one or more of the plurality of strut connections have an open eyelet (32, 32', 34, 34', 37)..

## Patentansprüche

1. Stent (10) zur Implantation in ein Körpergefäß, wobei der Stent Folgendes umfasst:
zumindest zwei Umfangselemente (15), die jeweils erste und zweite Längsenden (11, 12) und einen Längsachse (14) aufweisen,
eine Vielzahl von Strebenverbindungen (30, 31, 31', 33, 35, 36, 38), die am ersten Ende geformt sind;
eine Vielzahl von Strebenverbindungen (30, 31, 31', 33, 35, 36, 38), die am zweiten Ende geformt sind;
eine Vielzahl von bogenförmigen Streben (20), die bogenförmig (60, 62, 64, 70, 72, 74) relativ zu einer die Längsachse aufweisenden Ebene und zwischen den ersten und zweiten Enden im Abstand angeordnet sind;
worin die benachbarten Streben Ende an Ende durch zumindest eine Strebenverbindung verbunden sind und worin die Umfangselemente durch ein oder mehr Verbindungselemente (16) zur Bildung eines röhrenförmigen Hauptkörpers (110) miteinander verbunden sind, **dadurch gekennzeichnet, dass** jedes Verbindungselement das erste Längsende eines Umfangselements mit entweder dem ersten oder dem zweiten Längsende eines zweiten Umfangselements verbindet.

2. Vorrichtung nach Anspruch 1, worin jede Strebe einen ersten und einen zweiten Endabschnitt (22, 23) aufweist, der den ersten und zweiten Ende eines Umfangselements entspricht, worin zumindest eine der Streben konvex (25) ist, wodurch ein Zwischenabschnitt (26) der Strebe zwischen den ersten und zweiten Endabschnitten näher an der Längsachse liegt als die Endabschnitte.

3. Vorrichtung nach Anspruch 1, worin jede Strebe einen ersten und einen zweiten Endabschnitt (22, 23) aufweist, der den ersten und zweiten Ende eines Umfangselements entspricht, worin zumindest eine der Streben konkav (24) ist, wodurch ein Zwischenabschnitt der Strebe zwischen den ersten und zweiten Endabschnitten weiter von der Längsachse entfernt ist als die Endabschnitte.

4. Vorrichtung nach einem der vorhergehenden Ansprüche, worin der röhrenförmige Hauptkörper eine Längsachse (14) und erste und zweite Längsenden (111, 112) aufweist und ein mit Öffnungen (17, 18) an den ersten und zweiten Enden in Verbindung stehendes Lumen (13) definiert, wobei der Hauptkörper eine Konfiguration mit einem radial komprimierten ersten Durchmesser und eine Konfiguration mit einem radial expandierten zweiten Durchmesser aufweist.

5. Vorrichtung nach Anspruch 4, worin die expandierte Konfiguration ein z-förmiges Zickzackmuster umfasst.

6. Vorrichtung nach einem der vorhergehenden Ansprüche, worin das Umfangselement (15) oder der röhrenförmige Hauptkörper (110) mit einem Ballon expandiert werden kann.

7. Vorrichtung nah einem der Ansprüche 1 bis 5, worin das Umfangselement (15) oder ein röhrenförmiger Hauptkörper (110) selbst-expandierbar ist.

8. Vorrichtung nach einem der Ansprüche 1 bis 7, worin der Körper (110) teilweise selbst-expandierbar und teilweise ballon-expandierbar ist.

9. Vorrichtung nach einem der vorhergehenden Ansprüche, worin eine der Strebenverbindungen eine Kopfstrebenverbindung (33) umfasst.

10. Vorrichtung nach einem der vorhergehenden Ansprüche, worin zumindest eine der Strebenverbindungen eine Spulenkonstruktion (31, 32) umfasst.

11. Vorrichtung nach einem der vorhergehenden Ansprüche, worin zumindest eine der Strebenverbindungen eine Biegung (35, 36, 38) aus einem Material umfasst, das als diskreter Befestigungspunkt zur Fixierung des Stents an der Gefäßwand dient.

12. Vorrichtung nach einem der vorhergehenden Ansprüche, worin ein oder mehr der Vielzahl von Strebenverbindungen eine geschlossene Öse (32, 32') aufweisen.

13. Vorrichtung nach einem der vorhergehenden Ansprüche, worin ein oder mehr der Vielzahl von Strebenverbindungen eine offene Öse (32, 32', 34, 34', 37) aufweisen.

## Revendications

1. Stent (10) destiné à être implanté dans un vaisseau corporel, le stent comportant :
au moins deux éléments (15) circonférentiels, chacun présentant une première et une seconde extrémités longitudinales (11, 12) et un axe longitudinal (14),
une pluralité de connexions (30, 31, 31', 33, 35, 36, 38) d'entretoises formées au niveau de la première extrémité ;
une pluralité de connexions (30, 31, 31', 33, 35, 36, 38) d'entretoises formées au niveau de la seconde extrémité ;
une pluralité d'entretoises (20) arquées espacées d'une manière arquée (60, 62, 64, 70, 72, 74) par rapport à un plan comprenant l'axe longitudinal et disposées à un niveau intermédiaire entre les première et seconde extrémités ;
dans lequel les entretoises adjacentes sont jointes bout-à-bout par au moins une connexion d'entretoise, et dans lequel lesdits éléments circonférentiels sont joints par un ou plusieurs organes (16) de liaison pour former un corps (110) principal tubulaire, **caractérisé en ce que** chaque organe de liaison relie la première extrémité longitudinale d'un élément circonférentiel à la première ou la seconde extrémité longitudinale d'un second élément circonférentiel.

2. Dispositif selon la revendication 1 dans lequel chaque entretoise présente une première et une seconde parties (22, 23) d'extrémité correspondant aux première et seconde extrémités d'un élément circonférentiel dans lequel au moins une des entretoises est convexe (25), une partie intermédiaire (26) de l'entretoise entre les première et seconde parties d'extrémité étant plus près de l'axe longitudinal que les parties d'extrémité.

3. Dispositif selon la revendication 1 dans lequel chaque entretoise présente une première et une seconde parties (22, 23) d'extrémité correspondant aux première et seconde extrémités d'un élément circonférentiel dans lequel au moins une des entretoises est concave (24), une partie intermédiaire de l'entretoise entre les première et seconde parties d'extrémité étant plus loin de l'axe longitudinal que les parties d'extrémité.

4. Dispositif selon l'une quelconque des revendications précédentes dans lequel le corps tubulaire principal possède un axe (14) longitudinal et une première et seconde extrémités (111, 112) longitudinales et délimite une lumière (13) en communication avec des ouvertures (17, 18) au niveau des première et seconde extrémités, le corps principal ayant une première configuration avec le diamètre comprimé radialement et une seconde configuration avec le diamètre dilaté radialement.

5. Dispositif selon la revendication 4, dans lequel la configuration dilatée comporte un motif en forme de zigzag.

6. Dispositif selon l'une quelconque des revendications précédentes dans lequel l'élément (15) circonférentiel ou le corps (110) principal tubulaire est expansible par ballonnet.

7. Dispositif selon l'une quelconque des revendications 1 à 5 dans lequel l'élément (15) circonférentiel ou le corps (110) principal tubulaire est auto-expansible.

8. Dispositif selon l'une quelconque des revendications 1 à 7 dans lequel le corps (110) est partiellement auto-expansible et partiellement expansible par ballonnet.

9. Dispositif selon l'une quelconque des revendications précédentes dans lequel une des connexions d'entretoise comporte une connexion (33) d'entretoise de tête.

10. Dispositif selon l'une quelconque des revendications précédentes dans lequel au moins une des connexions d'entretoise comporte une structure (31, 32) enroulée.

11. Dispositif selon l'une quelconque des revendications précédentes dans lequel au moins une des connexions d'entretoise comporte une partie courbe (35, 36, 38) de matériau qui sert de point d'attache discret pour fixer le stent à la paroi du vaisseau.

12. Dispositif selon l'une quelconque des revendications précédentes dans lequel une ou plusieurs connexions d'entretoise parmi ladite pluralité de celles-ci sont munies d'un oeillet (32, 32') fermé.

13. Dispositif selon l'une quelconque des revendications précédentes dans lequel une ou plusieurs connexions d'entretoise parmi ladite pluralité de celles-ci sont munies d'un oeillet (32, 32', 34, 34', 37) ouvert.
